## Europäisches Patentamt

## European Patent Office

## Office européen des brevets

(19)

(11) Numéro de publication: **0 173 619**
**B1**

(12) **FASCICULE DE BREVET EUROPEEN**

(45) Date de publication du fascicule du brevet:
24.10.90

(21) Numéro de dépôt: 85401620.1

(22) Date de dépôt: 08.08.85

(51) Int. Cl.⁵: **C12N 15/00, C12N 1/00,**
**C12N 1/18, C12N 9/02,**
**C12P 21/02**

(54) Procédé de préparation d'une souche, notamment de levure, transformée par un vecteur d'expression, qui peut être cultivée sur un milieu complet sans pression de sélection et souche ainsi obtenue.

(30) Priorité: 09.08.84 FR 8412598

(43) Date de publication de la demande:
05.03.86 Bulletin 86/10

(45) Mention de la délivrance du brevet:
24.10.90 Bulletin 90/43

(84) Etats contractants désignés:
AT BE CH DE FR GB IT LI LU NL SE

(56) Documents cités:
EP-A- 0 011 562

**MOLECULAR AND GENERAL GENETICS,**
vol. 176, no. 3, 16 novembre 1979, pages 335-342, Springer-Verlag, DE; H. BLANC et al.: "Stable yeast transformation with chimeric plasmids using a 2 mum-curcular DNA-less strain as a recipient" CURRENT TOPICS IN MICROBIOLOGY AND IMMUNOLOGY, vol. 96, 1982, pages 119-144; C.P. HOLLENBERG: "Cloning with 2-mum DNA vectors and the expression of foreign genes in saccharomyces cerevisiae"

(73) Titulaire: TRANSGENE S.A., 16 rue Henri Regnault,
F-92400 Courbevoie(FR)

(72) Inventeur: Loison, Gérard, 1, rue de l'Aimant,
F-67000 Strasbourg(FR)

(74) Mandataire: Martin, Jean-Jacques et al, Cabinet REGIMBEAU 26, Avenue Kléber, F-75116 Paris(FR)

ACTORUM AG

## Description

La présente invention se rapporte au domaine des biotechnologies, en particulier à l'utilisation de cellules, notamment des levures telles que Saccharomyces cerevisiae, comme hôte d'expression de gènes à intérêt commercial.

Plus particulièrement, la présente invention concerne un moyen de cultiver et de maintenir des souches de levure à plasmide hybride sur n'importe quel type de milieu compatible avec le croissance des cellules non transformées, sans qu'il y ait perte du plasmide ou du phénotype allié.

Pour obtenir une expression maximale d'un gène, étranger ou non, cloné dans une levure telle que Saccharomyces cerevisiae, on cherche à ce que ce gène soit présent à un nombre d'exemplaires le plus élevé possible. Le moyen le plus évident et de fait généralement utilisé est de perpétuer le gène en l'intégrant dans un plasmide présent en multiples copies dans la cellule. Cependant, la transmission de ces plasmides hybrides de génération en génération n'est pas toujours assurée et divers systèmes de sélection ont été développés dans le but de ne permettre la prolifération que des cellules contenant le plasmide.

La plus courante de ces sélections, et la moins coûteuse car elle ne nécessite l'adjonction d'aucune drogue ou antibiotique dans le milieu de culture, consiste à prendre comme organisme hôte une souche présentant une auxotrophie consécutive à la mutation d'un gène donné et à intégrer dans le plasmide hybride un gène capable d'assurer la fonction déficiente.

Sur milieu minimum, seules les cellules contenant du plasmide hybride peuvent pousser alors que sur milieu complet toutes les cellules peuvent pousser y compris celles dépourvues de plasmide. L'inconvénient de ce système de sélection est qu'il restreint le choix du milieu de culture puisque celui-ci doit être dépourvu de toute molécule susceptible de complémenter l'exigence auxotrophique. En pratique, le milieu de culture le plus largement utilisé est un milieu synthétique de laboratoire, de prix assez coûteux.

La présente invention concerne un procédé pour rendre les souches de levure telles que Saccharomyces cerevisiae totalement dépendantes de la présence du plasmide pour leur survie, et ce quelle que soit la composition du milieu de culture.

On peut par cette invention faire pousser les cellules sur un milieu riche, ce qui présente de nombreux avantages : temps de génération plus court (croissance plus rapide d'environ 25 %), phase exponentielle de croissance beaucoup plus longue, masse totale de cellules en poids sec plus importante, etc.

Cette invention permet, d'autre part, d'utiliser tout autre milieu que ceux utilisés en laboratoire, en particulier des milieux utilisés en industrie tels que mouts d'origine variée, mélasse, etc., de coût beaucoup moins élevé.

On peut envisager de modifier les souches pour pouvoir les cultiver sur une variété de milieux non-définis et de coût minimal dans l'optique de leur faire produire des substances à intérêt industriel.

C'est pourquoi la présente invention concerne un procédé de préparation d'une souche transformée par un vecteur de clonage ou d'expression d'un gène codant pour une protéine déterminée qui peut être cultivée sur un milieu complet sans pression de sélection, procédé caractérisé en ce que :

(a) on identifie un métabolite dont l'absence est léthale pour la souche et qui est synthétisé par au moins deux voies métaboliques,

(b) on bloque une première desdites voies métaboliques en altérant au moins l'un des gènes impliqués dans ladite voie,

(c) on transforme la souche par le vecteur qui comporte, en outre, l'allèle fonctionnel du gène altéré,

(d) on bloque les autres voies métaboliques.

Il est bien entendu que la présente description sera effectuée essentiellement en se référant à des souches de levure mais d'autres souches peuvent être mises en oeuvre, en particulier des bactéries.

Les levures telles que S. cerevisiae ont deux voies métaboliques conduisant à la synthèse de l'uridine 5' monophosphate (UMP). La première est une voie dite de novo qui débute par la synthèse de carbamylphosphate et aboutit à l'UMP par transformation en 5 étapes dont les intermédiaires sont dans l'ordre l'acide uréidosuccinique, l'acide dihydroorotique, l'acide ortique et l'orotidine- 5'-monophosphate (OMP) (Lacroute, 1968; Jund et Lacroute, 1972).

La décarboxylation de l'OMP en UMP, dernière étape de la voie métabolique, est catalysée par une OMP décarboxylase dont le gène correspondant est appelé URA3 chez S. cerevisiae. Les souches ura3- dépourvues de cette activité OMP décarboxylase ne peuvent plus synthétiser l'UMP par la voie de novo, il leur faut une source de pyrimidine exogène, soit de l'uracile, soit de la cytosine, qui pourra être transformée en UMP grâce à la deuxième voie métabolique.

L'uracile et/ou la cytosine présents dans le milieu de culture peuvent rentrer dans la cellule grâce à une perméase spécifique pour chacune des deux molécules. Une fois dans la cellule, la cytosine est directement transformée en uracile, l'uracile est transformé en UMP grâce à une UMP pyrophosphorylase (appelée aussi uracile ribosyl phosphotransférase) (Jund et Lacroute, 1970).

Il n'existe pas d'autre voie métabolique importante qui conduise à l'UMP sans passer par l'OMP ou l'uracile, si bien que la cellule de levure ne peut survivre que si l'une au moins des deux voies décrites plus haut et résumées sur le schéma 1 fonctionne.

Le principe du procédé dans ce cas est le suivant :

On bloque la première voie métabolique en altérant un gène impliqué dans la synthèse d'une OMP décarboxylase active, c'est-à-dire en général le gène URA3, et on transforme la souche par un vecteur plasmidique comportant un gène codant pour une OMP décarboxylase active, c'est-à-dire en général le gène URA3.

De façon simple, on transforme un mutant ura3 par le plasmide approprié et on sélectionne les transformants ura⁺.

SCHEMA 1 : LES DEUX VOIES DE BIOSYNTHESE DE
L'URIDINE-5'-MONOPHOSPHATE  CHEZ LA
LEVURE S. cerevisiae

glutamine
$+CO_2+ATP$

↓

carbamylphosphate

↓

acide uréidosuccinique

↓

orotate

↓

orotidine-5'-monophosphate

(OMP)

étape catalysée par
l'OMP décarboxylase,
codée par le gène URA3

⇓

URIDINE-5'-MONOPHOSPHATE

(UMP)

étape catalysée par
l'UMP pyrophosphorylase
codée par le gène FUR1

cytosine ⟶ uracile

⟋⟍ voie
de
NOVO

voie
de
secours

membrane
cellulaire

cytosine du                     uracile du
milieu extérieur           milieu extérieur

4

On altère ensuite un gène impliqué dans la synthèse d'une UMP pyrophosphorylase active, c'est-à-dire un gène situé dans le locus FUR1.

Le gène codant pour l'UMP pyrophosphorylase de S. cerevisiae est appelé FUR1 (Jund et Lacroute, 1970), c'est un locus complexe dont certaines mutations résultent dans la résistance des cellules à toute une gamme d'analogues fluorés des bases pyrimidiques ou des nucléosides et nucléotides correspondants. L'effet de résistance le plus marqué résulte de l'absence de l'activité UMP pyrophosphorylase, le phénotype correspondant est récessif.

L'altération du gène FUR1 peut se faire par croisement avec un mutant caractérisé fur1, soit par mutation spontanée avec sélection des mutants sur un milieu approprié décrit dans un des exemples qui suit.

Il est bien entendu que le procédé de la présente invention est utile de façon générale pour préparer ou cloner une protéine quelconque qui est codée par le plasmide vecteur. Dans le cas d'une expression le plasmide comportera en outre les éléments nécessaires à l'expression du gène, en particulier un promoteur de levure tel que le promoteur de PGK (phosphoglycérate kinase) de levure et éventuellement un terminateur, par exemple le terminateur de PGK.

Le plasmide d'expression pourra, comme cela est décrit, comporter d'autres éléments permettant la construction dans une bactérie comme E. coli : origine de réplication, gène de résistance pour la sélection.

A titre d'exemple, on a pu exprimer le gène xylE de Pseudomonas putidae, mais il est bien entendu possible d'exprimer un gène quelconque.

En outre, il a été possible d'exprimer le gène codant pour l'$\alpha_1$-antitrypsine humaine.

EXEMPLE 1 : INTRODUCTION DE LA MUTATION fur1 DANS LE GENOME D'UNE SOUCHE DE LEVURE ura3 RENDUE ura+ PAR LA PRESENCE D'UN PLASMIDE

a) Construction du plasmide pTG807

Dans cet exemple, on a utilisé le plasmide pTG807 pour complémenter la déficience en activité OMP décarboxylase d'une souche ura3. Le plasmide pTG807 est un plasmide dérivé de pBR322 contenant au site HindIII un fragment (1,1 kilopaires de base) de DNA chromosomique de S. cerevisiae qui renferme le gène URA3 (allèle de type sauvage, Bach et al, 1979). A environ 80 paires de bases en aval de la fin (côté 3') du gène URA3, soit à environ 100 paires de base d'une extrémité du morceau cloné, se trouve un site unique SmaI. Ce site a servi à cloner un fragment de DNA d'environ 1 kilopaire de base délimité par les sites AvaII et HindIII de part et d'autre de l'origine de réplication du plasmide 2 microns (2 μ) - forme B de levure (Broach, 1981). Le fragment AvaII - HindIII a été obtenu par double digestion du DNA plasmidique de pFLI (Chevallier et al., 1979) séparation des différents fragments par électrophorèse en gel d'agarose, élution du fragment recherché par la technique décrite par Girvitz et al. (1980). Ce fragment a été ensuite traité à la DNA-polymérase 1 Klenow de E. coli en présence de dNTPs, puis à la DNA-ligase du phage T4 en présence d'ATP et du DNA de pTG802 préalablement linéarisé au site SmaI. On a pu ainsi obtenir deux nouveaux types de plasmide (pTG806 et pTG807) résultant de l'intégration du fragment AvaII-Hind III du 2 μ au site SmaI de pTG802 et ne différant entre eux que par l'orientation de fragment.

Le plasmide pTG807 (schéma 2) possède donc :
- une origine de réplication procaryotique fonctionnant dans E. coli,
- une origine de réplication eucaryotique fonctionnant dans S. cerevisiae,
- un gène (ApR) résistance à l'ampicilline permettant la sélection dans E. coli et la détection des transformés de S. cerevisiae (test de Chevallier et Aigle,1979),
- le gène URA3 codant pour l'OMP décarboxylase et permettant la sélection du plasmide à la fois sans S. cerevisiae (mutant ura3 et dans E. coli (mutant pyrF) (Bach et al, 1979).

Le fait que l'on ait cloné l'origine de réplication du plasmide 2 μ de levure sang le locus REP3 (Broach, 1983) dans le plasmide pTG807 rend très instable la présence de ce plasmide dans les cellules de levure qui en se divisant génèrent des cellules pouvant ne plus contenir le plasmide hybride (ségrégation mitotique). Lors d'une culture liquide en milieu sélectif, on observe un état d'équilibre entre les cellules qui renferment le plasmide - capables, par conséquent, de former des colonies après étalement sur milieu sélectif solide - et celles qui ne le renferment plus - incapables de former des clones sur milieu sélectif solide. La très grande fréquence de perte et le fait que les cellules venant de perdre le plasmide gardent la capacité à se diviser un nombre restreint de fois, font que cet état d'équilibre, même dans une culture en phase exponentielle de croissance en milieu sélectif, reste en faveur des cellules ayant perdu le plasmide (60 % dans ce cas environ). Pour d'autres plasmides hybrides ayant à la fois l'origine de réplication et le locus REP3 du 2 μ, ce rapport peut varier de moins de 5 % (cas de pJDB207 - Beggs, 1981) à 25 % ou plus. Dans cet exemple, on a choisi le cas extrême d'une grande instabilité phénotypique liée à la ségrégation mitotique du plasmide pour faciliter l'étude. On verra au cours d'autres exemples le cas où la ségrégation est moins importante (cas plus favorable où le plasmide sert à exprimer des gènes étrangers à intérêt industriel).

SCHEMA 2 - STRUCTURE DU PLASMIDE pTG807

SmaI°/HindIII°        AvaII°/SmaI°

URA3⁺        Tet.ᴿ        Amp.ᴿ

oriS        oriE

HindIII        HindIII

☐ séquence de DNA chromosomique de S. cerevisiae

─── séquence de DNA de pBR322

- - - - séquence de DNA du plasmide 2μ

■ région contenant l'origine de réplication fonctionnelle dans E. coli (oriE)

región contenant l'origine de réplication fonctionnelle dans S. cerevisiae (oriS)

1 Kb        échelle

b) Etude de l'effet de la mutation fur1 sur le phénotype plasmidique des souches transformées

On possède une souche de laboratoire S. cerevisiae ura⁻ TGY10-1 (Mata ura3-251-273-328). Toute autre souche de génotype ura3⁻ peut être utilisée. Cette souche a été rendue ura⁺ par transformation avec le DNA de pTG807 selon le technique décrite par Hinnen et al. (1978). La souche transformée TGY10-1/pTG807 a été croisée avec la souche isogénique FE 25-3 (Matα fur1-8) pour former le diploïde TGY7 (cf. Mortimer et Hawthorne 1966). Au bout de 8 heures sur milieu complet YPG, le mélange de co-

6

pulation a été transféré 24 heures sur milieu de présporulation (Glucose 0,1 %, acétate de potassium 1,1 M; Yeast extract Difco 0,25 %, sulfate d'ammonium 5 g/l, Agar Difco 2 %) puis 48 heures sur milieu de sporulation (acétate de potassium 1 %, agar Difco 2 %). La formation des asques étant jugée suffisante après observation au microscope, on a traité le mélange de copulation à l'éther qui tue préférentiellement les cellules et au moins 75 % des spores. Le traitement à l'éther se fait de la façon suivante :

Le contenu d'une anse de platine en mélange de copulation est resuspendu dans 1 ml d'eau. Après addition d'1 ml d'éther, on agite vigoureusement pendant 5 min et cela deux ou trois fois en l'espace d'une demi-heure. On étale, 0,1 ml de la phase aqueuse pure et 0,1 ml diluée 10 fois chaque fois sur une boite de milieu complet (YPG). On sélectionne ainsi les souches haploïdes ségrégeant du croisement.

Dix-sept colonies ainsi obtenues ont été reprises, cultivées sur milieu complet solide, et testées pour leur capacité à produire la β-lactamase selon la technique de Chevallier et Aigle (1979). Sur 17 colonies, 4 seulement produisaient la β-lactamase.

On a testé les signes sexuels de ces 4 souches productrices, on a conservé une de ces souches de signe a, pour la poursuite de l'étude. Cette souche est appelée TGY7-11. L'analyse de la résistance des cellules TGY7-11 à différents analogues de l'uracile tels que 6-aza Uracile et 5-Fluorouracile a permis de montrer que cette souche présentait la mutation fur1-8 (tableau I ci-après).

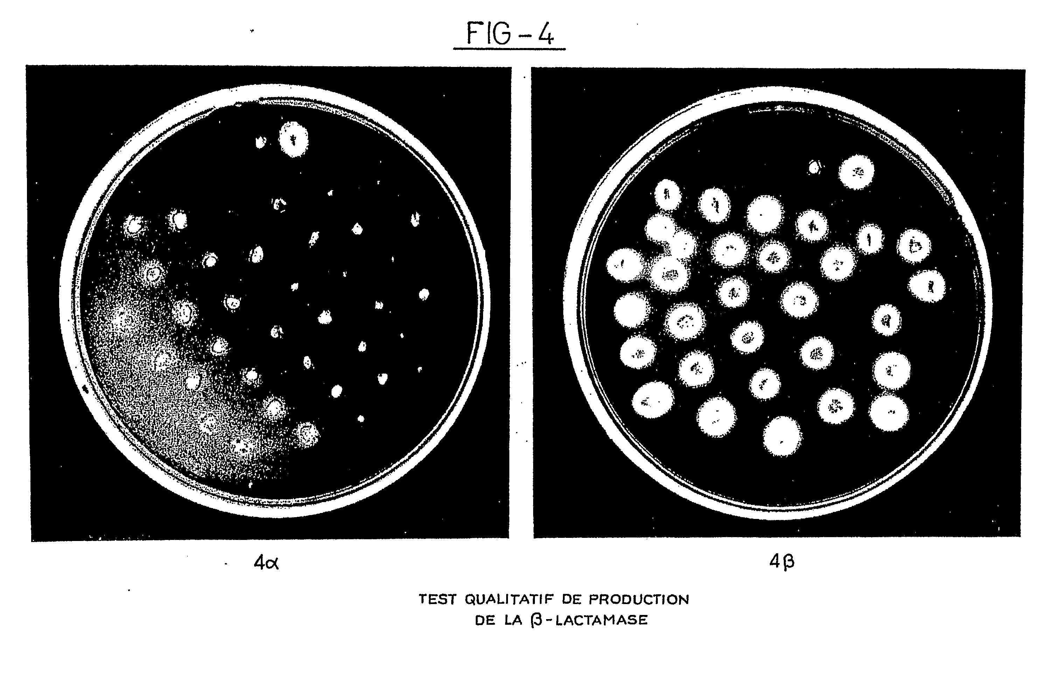

La souche TGY7-11/pTG807 a été repiquée en milieu complet liquide (YPG) de la façon suivante : le soir on a ensemencé 25 ml de milieu avec $10^4$ cellules/ml, laissé pousser la nuit jusqu'à obtenir environ $10^7$ cellules/ml (10 générations) puis on a recommencé un ensemencement identique à partir de cette culture et cela deux fois de suite de façon à obtenir entre 30 et 40 générations cellulaires en milieu complet. Les cellules ont alors été diluées et étalées sur boîte de milieu complet de façon à obtenir environ 200 colonies isolées par boîte (figure 4).

La perte du plasmide a été testée sur une trentaine de colonies par la technique de Chevallier et Aigle (1979) (figure  ) en guise de contrôle, l'expérience a été réalisée en parallèle avec la souche parentale TGY10-1/pTG807 cultivée dans les mêmes conditions.

Une trentaine de colonies ont été repiquées en petits dépôts sur une boîte de Pétri contenant un milieu renfermant de l'amidon (Yeast nitrogen base DIFCO 6,5 g/l ; glucose 1 g/l ; amidon soluble 2 g/l , uracile 30 mg/l ; agar 20 g/l tamponné à pH6 par du tampon phosphate 0,02 M). Après 24 heures d'incubation à 30°C, on coule une couche du même milieu en surfusion (44°C, 10 g/l d'agar) dans lequel on a ajouté 1,5 ml de réactif (3 mg/ml I2 ; 150 mg/ml IK, 3 mg/ml ampicilline). Les boîtes sont incubées une nuit au réfrigérateur puis à l'obscurité à température ambiante. Autour des souches productrices se développe un halo de décoloration. Sur le cliché 4α (figure 4), on observe la culture de colonies issues de la souche TGY-10-1 transformée par pTG807. La première rangée, en haut, concerne deux contrôles; l'un négatif, à gauche, il s'agit d'une colonie TGY 10-1 non transformée ; l'autre positif, à droite, représente TGY 10-1/pTG807 maintenu en milieu sélectif. De même sur le cliché 4β, on observe la culture de colonies issues de la souche TGY 7-11 (ura3. fur1) transformée par pTG807. De façon identique qu'en 4α, deux contrôles ont été effectués sur la première rangée en haut.

Toutes ces colonies de la souche TGY7-11 ainsi cultivées présentent le plasmide pTG807, alors qu'aucune de la souche TGY10-1 ne présente ce plasmide.

Les deux souches TGY10-1 et TGY7-11 étant isogéniques, elles ne diffèrent que par la mutation fur1-8 présente dans TGY7-11. On peut donc conclure que la présence de cette mutation rend la souche TGY7-11 incapable de survivre sans plasmide sur milieu complet.

RESISTANCE DE DIFFERENTES SOUCHES
DE S. cerevisiae A L'EFFET TOXIQUE DU
5-FLUORO-URACILE

| Souche | Concentration maximale de 5-fluoro-uracile tolérée par les cellules |
|---|---|
| TGY10-1 | $<3 \cdot 10^{-5}$ M |
| FE25-3 | $10^{-2}$ M |
| TGY7-11 pTG807 | $10^{-2}$ M |

Le seuil de résistance à l'effet toxique du 5-fluorouracile a été déterminé sur boîte de Pétri contenant du milieu minimum supplémenté avec de l'uracile (30 µg/ml) et des quantités croissantes de l'analogue fluoré ($10^{-6}$ M et plus). Les concentrations maximales reportées plus haut sont celles au dessus desquelles les cellules ne poussent plus.

TABLEAU I

EXEMPLE II : EXPRESSION D'UN GENE ETRANGER DANS UNE SOUCHE DE LEVURE ura3 fur1.

Dans cet exemple on montre :
- qu'il est possible de cultiver sur milieu complet une souche S. cerevisiae ura3 fur1 transformée par un plasmide hybride sans risquer la perte du phénotype lié au plasmide,
- qu'une telle souche cultivée dans ces conditions est capable d'exprimer un gène étranger porté par le plasmide,
- que la mutation fur1 ne gène pas le niveau d'expression du gène étranger.

a) Construction d'une souche diploïde comme réceptrice de transformation

Les souches S. cerevisiae ura3 fur1 dépourvues à la fois des activités OMP décarboxylase et UMP pyrophosphorylase ne sont pas viables. On ne peut donc les utiliser comme réceptrices de transformation. Il faut donc transformer en un premier temps une souche ura3 en ura+ par le biais d'un plasmide puis rendre cette souche fur1 soit par croisement comme décrit dans l'exemple I, soit par sélection (exemple III). Une alternative à la première de ces possibilités est de transformer une souche diploïde homozygote pour l'allèle muté ura3 et hétérozygote pour fur1. Une fois rendue ura+ par transformation plasmidique, on peut soit faire sporuler la souche et sélectionner les ségrégeants haploïdes _fur_1, ou alors sélection-ner directement des dérivés homozygotes pour _fur_1 (par conversion génique) de la souche diploïde et utiliser cette souche diploïde comme hôte d'expression. Il est très facile de sélectionner des convertis di-ploïdes _fur_1 sur milieu contenant du 5-fluorouracile ou du 6-azauracile du fait de la résistance accrue de ces souches à l'effet toxique de ces drogues.

La souche haploïde _S. cerevisiae_ TGY7-11 (Mata _ura_3 _fur_1-8/pTG807), dont la construction a été décrite dans l'exemple précédent, a été croisée avec une souche haploïde non apparentée, dérivé _ura_3 iso-génique de TGY100-1 (Matα, _his_3, _leu_2). On a isolé par micromanipulation un zygote diploïde résultant de ce croisement dont le génotype est

$$\left(\frac{ura3}{ura3}\ \frac{his3}{+}\ \frac{leu2}{+}\ \frac{fur1}{+}\Big/pTG807\right)$$

et le phénotype sauvage. En cultivant cette souche sur milieu complet, il a été facile d'obtenir des colonies ayant perdu le plasmide pTG807 et donc devenues ura⁻. Une telle colonie a été isolée et la souche correspondante, appelée TGY8, a été stockée comme réceptrice de transformation.

La souche TGY8 a fait l'objet d'un dépôt auprès de la Collection Nationale de Cultures de Microorganismes de l'Institut Pasteur, portant le numéro d'ordre I-327.

b)Transformation de la souche TGY8 par un plasmide hybride portant le gène URA3 de S. cerevisiae et le gène xyl E de Pseudomonas putida

Le plasmide pTG843 est un dérivé du vecteur d'expression pTG833 dont la construction a été détaillée dans la demande de brevet français n° 8308292. En résumé, pTG843 a la structure suivante: la séquence de base est celle de pBR322. A la place du site _EcoR_I se trouve intégré le fragment qui comprend le gène _URA3_ et l'origine de réplication du plasmide 2μ et dont la construction a été expliquée à propos de pTG807 (exemple 2). Les séquences de pBR322 entre les sites _Hind_III et _Pvu_II ont été remplacées par le gène _xyl_ E de pseudomonas (brevets n° 16016/83 et n° 8308292) placé en sandwich entre le promoteur et le terminateur du gène _PGK_ de levure, de façon à ce que l'expression de _xyl_ E dans la levure se fasse sous le contrôle des signaux normalement utilisés pour exprimer le gène PGK (demande de brevet française n° 8402350).

Les levures transformées par le plasmide pTG843 synthétisent la catéchol 2.3-oxygénase qui oxyde le catéchol (produit incolore) en produit jaune. Les colonies de levure transformées par ce plasmide, lorsqu'on les vaporise avec du catéchol, prennent une coloration jaune.

La souche TGY8 a été transformée par pTG843 en ura⁺ selon la technique déjà décrite (Hinnen et al, 1978).

c) Expression du gène xyl E dans les souches ura3 fur1 pTG843 cultivées en milieu complet

La souche TGY8/pTG843 a été mise à sporuler comme décrit dans l'exemple I pour TGY7. Des ségrégeants haploïdes ont été isolés par le traitement éther comme décrit plus haut. Parmi ces ségrégeants ha-ploïdes, 5 souches _fur_1-8 ont été sélectionnées sur la base de leur résistance à l'effet toxique du 5-fluo-rouracide 5 10⁻³M.

Comme la souche TGY8 est issue de deux parents non isogéniques, sa descendance est constituée de souches haploïdes toutes génétiquement différentes les unes des autres. De fait, les 5 ségrégeants ha-ploïdes _fur_1- 8 présentent tous la coloration jaune après la vaporisation au catéchol, mais l'intensité et la rapidité de la réponse varient énormément d'une souche à l'autre, ce qui trahit la diversité génétique de ces souches.

Ces 5 souches appelées respectivement TGY8-2, TGY8-3, TGY8-7, TGY8-9 et TGY8-10 ont été cultivées pendant plus de trente générations en milieu complet comme décrit dans l'exemple I pour TGY7-11. En parallèle, une souche de laboratoire TGY1sp1 (_ura_3-_his_3) transformée par pTG843 a été cultivée dans les mêmes conditions. Les cultures ont ensuite été diluées puis étalées sur milieu complet de façon à obtenir une centaine de colonies isolées par boîte. Après trois jours de croissance les cellules ont été vaporisées au catéchol et répliquées sur milieu minimum complémenté en casaminoacides. Dans le cas des souches _ura_3 _fur_1, toutes les colonies ont répondu au catéchol en jaunissant, et toutes se sont avérées capables de pousser sur milieu minimum plus aminoacides. Dans le cas de la souche TGY1sp1, aucune coloration n'était observable; aucune colonie n'était capable de pousser sur milieu minimum plus aminoacides. Ces résultats confirment ceux exposés dans l'exemple I, à savoir que même sur milieu complet la mutation _fur_1-8 permet de contresélectionner les cellules ayant perdu le plasmide. Les 5 souches _ura_3 _fur_1 pTG843 et la souche TGY1sp1 pTG843 ont été cultivées une nuit en milieu liquide, complet pour les cinq premières, minimum plus histidine pour la dernière. Les cellules ont été recueillies le matin en phase exponentielle de croissance, broyées et testées pour leur contenu en catéchol-2-3-oxygénase. Le résultat du dosage est reporté sur le tableau II (test 1). L'activité spécifique de la catéchol-2-3-oxygénase de TGY1sp1 pMG843 étant arbitrairement fixée à 1, on voit que l'activité des souches ségrégeant de TGY8 varie de 6 à 19. Cette surproduction par rapport à l'expression dans TGY1 sp1 peut s'expliquer ou bien par une différence génétique entre les souches, ou bien par un effet des conditions de culture. Pour tester cette seconde hypothèse nous avons recommencé l'expérience avec la souche TGY8-3 cultivée en parallèle en milieu complet et milieu minimum. Le résultat, reporté dans le tableau II (test 2). montre que l'activité en catéchol-2-3-oxygénase est nettement meilleure de presque un facteur huit - lorsqu'on cultive les cellules sur milieu complet.

La souche TGY8-3 pTG843 a fait l'objet d'un dépôt auprès de la Collection Nationale de Cultures de Microorganismes de l'Institut Pasteur, portant le numéro d'ordre I-326.

## EXEMPLE III : SELECTION DE MUTANTS fur1 SPONTANES A PARTIR D'UNE SOUCHE ura3 TRANSFORMEE PAR UN PLASMIDE HYBRIDE

Un des paramètres impliqués dans le niveau d'expression d'un gène cloné dans la levure peut être le génome de la souche réceptrice. En supposant que l'on ait réussi à sélectionner une souche ura3 capable d'exprimer à un très haut niveau un gène étranger donné, il peut être désavantageux de la croiser avec une souche fur1 non-isogénique au risque de perdre le caractère de très bonne expression dans le diploïde ou dans la descendance. Il est en revanche possible de sélectionner directement des mutants fur1 spontanés à partir de la souche ura3 transformée. Cette deuxième stratégie a comme avantage de permettre la comparaison entre le mutant fur1 et la souche dont il dérive et de pouvoir ainsi étudier l'effet spécifique éventuel de la mutation fur1 sur la physiologie de la souche.

Dans cet exemple on a choisi la souche déjà mentionnée TGY1sp1 (Mata ura3 his3), cette fois-ci transformée par le plasmide pTG856. La structure de pTG856 a été décrite dans la demande de brevet français n° 8315716.Ce plasmide contient la séquence du gène URA3 ; l'origine de réplication du plasmide 2 μ avec le locus REP3 qui diminue fortement la ségrégation mitotique des plasmides hybrides (Jayaran et al., 1983) ; Kikuchi, 1983 ; ce dernier auteur désigne ce locus sous le nom de STB) ; la séquence codante du gène de la glycoprotéine rabique flanquée du promoteur, en amont, et du terminateur, en aval, du gène PGK de levure ; et des séquences de pBR322 dont l'origine de réplication et le gène de résistance à l'ampicilline.

La souche TGY1sp1 pTG856 doit être cultivée sur milieu minimum supplémenté en histidine, mais pas sur milieu complet où les cellules tendent à perdre leur capacité à produire la glycoprotéine rabique. Pour permettre la culture de la souche sur milieu riche, on peut sélectionner des mutants fur1 sur la base de leur résistance à l'effet toxique d'analogues des bases pyrimidiques, tels que le 6-Azauracile, le 5-Fluorouracile, la 5-fluorocytosine, etc..

Environ $10^7$ cellules de la souche TGY1 sp1 pTG856 ont été étalées sur milieu complet additionné de 5-Fluorouracile = 5-Fu 5 mM. Après 8 jours d'incubation à 30°C, douze surcolonies ont été repiquées et subclonées sur milieu complet additionné de 5-Fluorouracile 1 mM. Après subclonage, les douze souches ont été mises à cultiver dans du milieu complet (1 ml), puis reprises en fin de phase exponentielle et diluées 1000 fois dans le milieu complet (1 ml) pour des cultures la nuit. Le lendemain les cellules ont été diluées et étalées sur boîte de milieu complet de façon à obtenir de chaque souche cent à deux cents colonies isolées. En parallèle, on a réalisé la même série de cultures avec la souche originelle TGYsp1/pTG856. La proportion de colonies ayant gardé la capacité à pousser sur milieu minimum plus histidine a été testée par replique en velours. Cette proportion est comprise entre 25 et 30 % pour la souche TG-Y1sp1 pTG856 et 8 dérivés résistant au 5-Fu, et est de 100 % pour 4 autres souches résistantes au 5-Fu. On a donc obtenu 4 souches sur 12 capables de pousser sur milieu complet sans perte du phénotype lié à la présence du plasmide, on a vérifié par un test de complémentation allélique que ces 4 souches présentaient une mutation de type fur1, alors que les 8 autres souches présentaient une ou des mutations à d'autre(s) locus que Fur1.

Cet exemple montre qu'on peut aisément obtenir des mutants fur1 à partir d'une souche ura3 transformée par un plasmide, et que de tels mutants peuvent être utilisés en culture dans du milieu riche.

TABLEAU II : DOSAGE DE LA CATECHOL-OXYGENASE

DANS DIFFERENTES SOUCHES DE LEVURE

Test 1 :

| Souche | TGY8-2 | TGY8-3 | TGY8-7 | TGY8-9 | TGY8-10 | TGY1 sp1 |
|--------|--------|--------|--------|--------|---------|----------|
| milieu | complet | complet | complet | complet | complet | min. + his. |
| Activité spécifique catéchol-oxygénase (mU/mg protéine) | 39(5.6) | 95(13.5) | 69(9.8) | 45(6.4) | 131(18.7) | 7(1) |

Test 2 :

| Souche | TGY8-3 | TGY8-3 |
|--------|--------|--------|
| milieu | complet | minimum |
| Activité spécifique catéchol-oxygénase (mU/mg protéine) | 79 | 10 |

Les chiffres indiqués entre parenthèses indiquent les rapports de surproduction par rapport à une souche $FUR1^+$ cultivée en milieu minimum+histidine.

EP 0 173 619 B1

## EXEMPLE IV - CONSTRUCTION D'UN PLASMIDE PERMETTANT L'EXPRESSION EFFICACE D'$\alpha_1$-ANTITRYPSINE HUMAINE DANS LA LEVURE

Un fragment d'ADN contenant le gène d'$\alpha_1$-antitrypsine ($\alpha_1$-AT) a été isolé à partir d'un plasmide utilisé pour la préparation de cette protéine dans des extraits d'E. coli (brevets FR 2539758 et 2594082 et brevet PCT/FR 84/00014). Le plasmide pTG929 a été décrit dans les brevets précédents. Cet ADN plasmidique a été digéré par Pst1 puis soumis à l'action exonucléasique de la Klenow en présence des 4 dNTPs et enfin digéré par BglII. Le fragment contenant le gène l'$\alpha_1$-AT a été alors isolé et inséré entre le site BglII et EcoRI (traité à la Klenow) du plasmide pTG834 (figure 1) dont la construction et les propriétés ont été décrites en détail dans le brevet FR 8407125. Le plasmide résultant (pTG964) a été isolé d'E. coli, vérifié en séquence puis digéré par BglII et PstI.

Le fragment contenant le gène de l'$\alpha_1$-AT a été transféré dans un autre vecteur d'expression de la levure pTG849 par remplacement d'un fragment BglII-PstI comme décrit dans la figure 2. La construction et la structure de pTG849 ont été décrits en détail dans le brevet FR 2552776. Le plasmide résultant est appelé pTG968.

## EXEMPLE V - OBTENTION D'UNE SOUCHE ura3 fur1 TRANSFORMEE PAR UN PLASMIDE

L'ADN de pTG968 a servi à transformer la souche de levure TGY8 décrite précédemment pour la non-exigence en uracile, la souche transformée TGY8 pTG968 a été induite à sporuler et les spores fur1 ont été sélectionnés pour leur résistance à l'effet toxique du 5 Fu comme décrit précédemment.

Une souche haploïde appelée TGY8sp33 dérivée d'une de ces spores fur1 a servi à l'étude qui suit.

Le génotype de la souche TGY8sp33 est :

Matα/Mata, ura3-251-373-328/ura3-852, fur1-8/+, leu2-3-12/+, his3-11-15/+.

## EXEMPLE VI - PRODUCTION D'$\alpha_1$-AT EN FERMENTEUR DANS UN MILIEU RICHE A BASE DE MELASSE

Des cultures en semi-discontinu ("feed batch") ont été réalisées en fermenteur de 2 l (Biolafitte). Le levain consiste en une culture de 12 heures (500 ml) de la souche TGY8sp33 dans du milieu YPG. 500 ml de culture sont mis dans le fermenteur. Une solution concentrée de milieu (mélasse de betterave : 100 g/l, $(NH_4)_2SO_4$ : 2 g/l, $KH_2PO_4$ : 2 g/l, $MgSO_4$, $7H_2O$ : 0,6 g/l) est ajoutée dans le fermenteur afin de maintenir la concentration du substrat carboné à un taux faible (200 mg/l). La température est maintenue à 30°C, le pH est régulé à pH = 5 par une solution d'ammoniaque à 20 g/l. L'aération varie entre 1 VVM et 0,3 VVM au cours de la culture. La tension d'oxygène dissous est maintenue à 20 % grâce à une vitesse d'agitation variable.

Au cours de la culture, l'éthanol est dosé par voie enzymatique (kit Boehringer). Les concentrations de glucose et de saccharose sont déterminées par un appareil "T accussel PRG - GLUC". La croissance des levures est suivie par mesure de la densité optique à 600 nm (spectrophotomètre Philips PYE UNI-CAM PU 8610) ainsi que par mesure des poids secs.

Afin d'évaluer la concentration d'$\alpha_1$-AT, 10 ml de culture sont centrifugés (centrifugeuse JOUAN) pendant 5 minutes à 5000 tours/minute, le culot est lavé une fois dans 10 ml de tampon phosphate 0,1 M, pH 7. Les levures sont reprises dans une quantité appropriée de tampon (0,4 ml), cassées avec des billes de verre (diamètre 0,45 mm) pendant 4 cycles de 1 minute. Les extraits sont centrifugés pendant 10 minutes à 10000 tours/minute (centrifugeuse Sigma 2MK) et le surnageant est utilisé pour l'évaluation de la quantité de protéines et d'$\alpha_1$-AT. La concentration d'$\alpha_1$-AT est déterminée par Radial-Immunodiffusion (LC Partigen - Behring). La concentration des protéines est déterminée suivant la technique de Bradford (1976) (Bio-Rad kit).

Le pourcentage de cellules prototrophes est déterminé après étalement de plusieurs dilutions de la culture sur des boîtes de Pétri contenant du milieu riche YPG). Après 2 jours d'incubation à 30°C, les levures sont répliquées sur des boîtes de Pétri contenant du milieu YNBG+casamino acides (0,5 %).

## TABLEAU III

| Souche | nombre de colonies testées | % de cellules exigeant de l'uracile |
|---|---|---|
| TGY1SP4 ura 3 | 2691 | 12 |
| TGY8sp33 ura 3 fur1 | 4018 | 0 |

Les cellules qui poussent en milieu riche mais qui sont inaptes à pousser en milieu minimum sont devenues exigentes pour l'uracile.

La culture en fermenteur de la souche ura3 fur1 transformée par un plasmide (gène de l'$\alpha_1$-AT) montre :

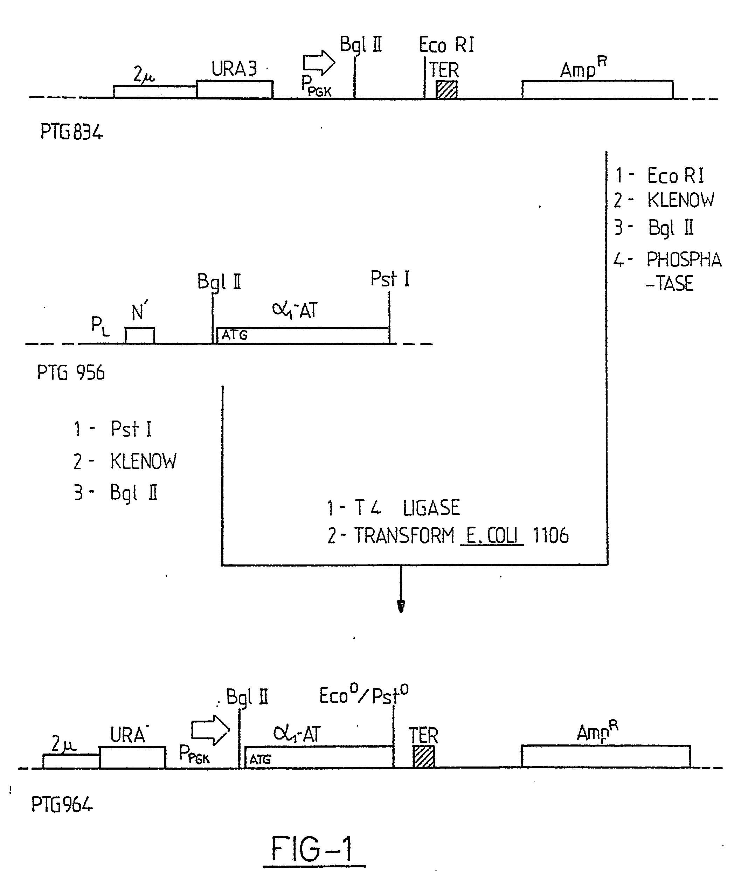

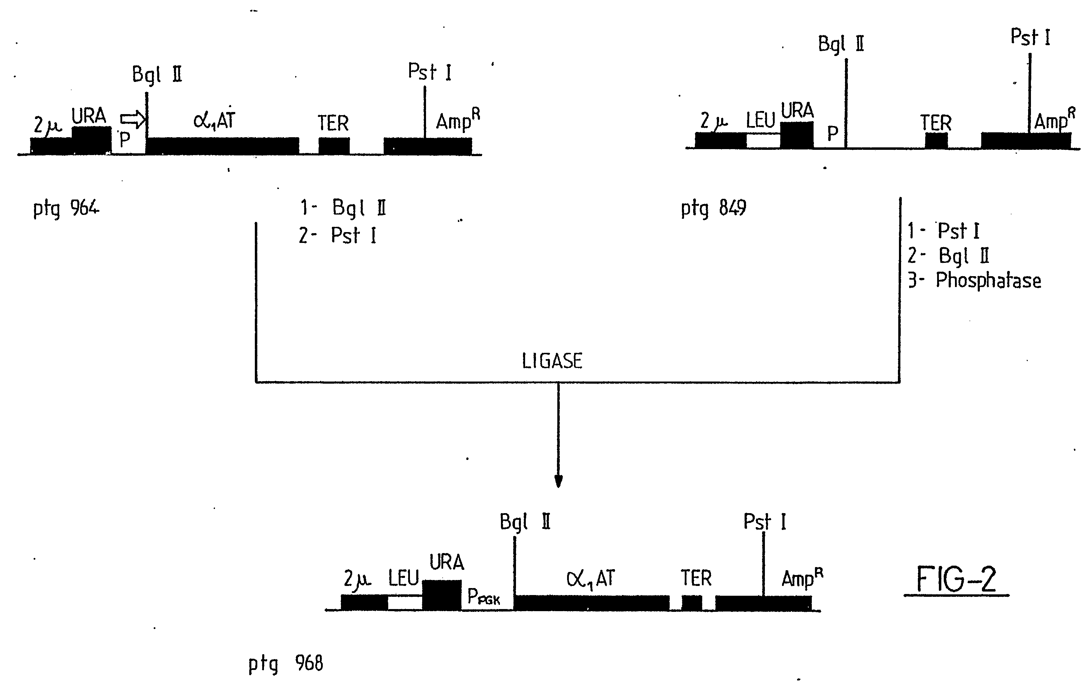

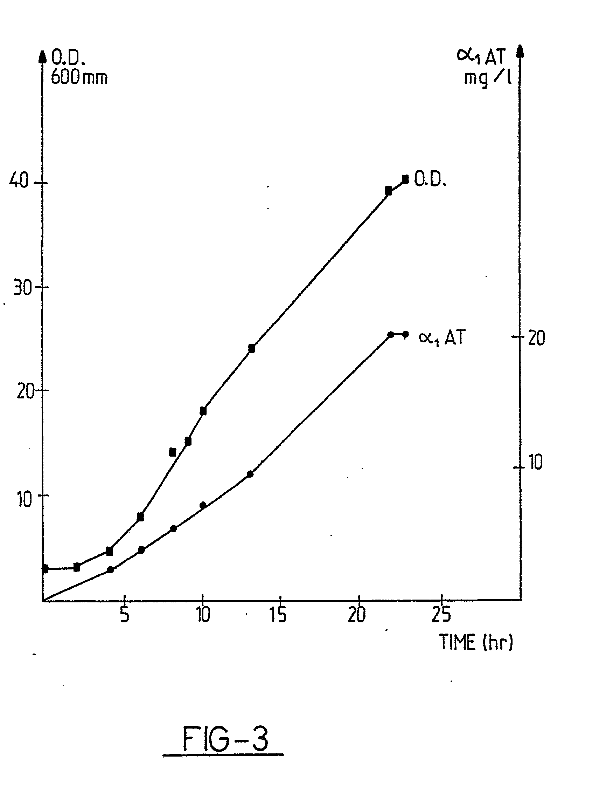

- que la production d'$\alpha_1$-AT est couplée à la croissance de la souche (figure 3)
- que le plasmide est stable (tableau III : aucune perte de plasmide n'est observée après 20 générations de culture, ce qui n'est pas le cas pour une souche ura3 (TGY1sp4).

EXEMPLE VII - STABILITE DE LA SOUCHE ura3 fur1 AU COURS D'UNE SERIE DE CULTURES EN ERLENMEYER

Afin de tester l'apparition de révertants fur+ au cours d'un nombre important de générations, on a procédé à diverses subcultures en milieu complet : un erlenmeyer contenant du milieu YPG (100 ml) est inoculé avec $10^6$ cellules et agité jusqu'à atteindre $10^8$ cellules/ml (12 générations). Les cellules sont alors diluées à $10^4$/ml dans une nouvelle culture de 100 ml ; l'opération est répétée jusqu'à 110 générations. Les cellules sont alors diluées à $7.10^3$ /ml et 7 aliquots de cette dilution (100 µl) sont répartis en boîtes de Pétri.

Environ 5000 colonies ont été obtenues, toutes ont poussé en milieu minimum et ont résisté au 5-fluorouracile (5 mM).

Un échantillon a également été utilisé pour le dosage de l'$\alpha_1$-AT. Aucun changement dans le niveau d'expression n'a été détecté (1 % de protéines totales).

Ces résultats indiquent qu'au bout de 110 générations de culture, la souche ura3 fur1 n'a pas subi de réarrangements importants.

Les expériences décrites montrent que la souche de levure ura3 fur1 peut être utilisée pour la production de protéines étrangères dans un milieu complexe, notamment celui qui est utilisé pour la production de levures.

EXEMPLE VIII - TRANSFORMATION D'UNE SOUCHE DE LEVURE ura3 PAR UN PLASMIDE PORTANT, ENTRE AUTRES, LE GENE URA3+ ET UN GENE CODANT POUR LA PRE-PRO-HIRUDINE

Description du plasmide pTG1818

Le plasmide pTG1818, décrit dans le brevet FR 8506672, est un plasmide-navette E. coli-S. cerevisiae conçu pour assurer l'expression de l'hirudine dans la levure et sa sécrétion dans le milieu de culture.

Il comprend des séquences du plasmide pBR322 de E. coli qui permettent, dans le colibacille, sa réplication et sa maintenance par sélection à l'ampicilline.

En outre, le plasmide pTG1818 possède un fragment du plasmide 2μ de levure (forme B) contenant l'ars principal et le locus STB (ou REP3) qui lui est lié.

Ce plasmide contient également le gène URA3 de levure. On peut donc maintenir pTG1818 dans la levure en utilisant des souches ura3, la sélection se faisant par complémentation sur milieu dépourvu de source de pyrimidine.

Les séquences nécessaires à l'expression de l'hirundine sont composées d'un fragment EcoRI-BglII d'environ 1,2 kb contenant la région 5' du gène du précurseur de la phéromone (y compris le promoteur) jusqu'au premier site HindIII (Kurjan et Herskowitz, 1982) fusionné à la séquence de l'hirudine de façon à obtenir la synthèse du précurseur : $NH_2$-Pre-Pro-Ser Leu Asp Glu Ala Glu Ala Ser Leu Asp-hirudine-COOH où Pre-Pro est composé de la séquence des 80 premiers aminoacides $NH_2$-terminaux de la phéromone de levure et hirudine représente la séquence de l'hirudine mature.

La souche TGY1sp4 (Matα his3-11-15 ura3-251- 373-328) transformée par pTG1818 sécrète une protéine qui présente une activité antithrombine typique de l'hirudine. Cette protéine représente près de 10 % des protéines que l'on trouve dans le milieu de culture (milieu minimum plus 0,5 % de casaminoacides ; culture de 10 ml en erlenmeyer de 50 ml ; à 30°C).

Stabilité du plasmide pTG1818 dans la souche TGY1sp4 cultivée en absence de sélection (milieu riche)

Pour tester la stabilité de pTG1818 dans les cellules TGY1sp4 cultivées en présence de source exogène de pyrimidine, on a cultivé la souche transformée TGY1sp4 pTG1818 dans 10 ml de milieu riche (YPG) de façon à obtenir environ 10 générations cellulaires (ensemencement à $10^4$ cellules/ml, culture de 20 heures). Les cellules ont alors été diluées et environ 200 cellules ont été étalées sur une boîte de milieu riche solide. Après 2 jours de croissance, les colonies ont été répliquées par velours sur milieu minimum + 0,5 % de casaminoacides. Environ 15 % des colonies se sont révélées incapables de croître sur ce dernier milieu, ce qui indique que les cellules ont perdu le plasmide.

Sélection de dérivés résistants au 5-FU à partir de TYG1sp4 pTG1818

Pour sélectionner de dérivés fur1 à partir de la TGY1sp4 pTG1818, on a étalé $2.10^6$ cellules de cette souche sur milieu complet contenant 0,3 mg/ml de 5-fluoro-uracile. Au bout d'une semaine d'incubation à 30°C une centaine de surcolonies était visible malgré la croissance résiduelle importante du tapis cellulaire. Six de ces surcolonies parmi les plus grosses ont été subclonées sur YPG + 5- FU 0,9 mg/ml. Sur les 6, 5 présentaient une bonne croissance tandis que la sixième offrait une moindre résistance à l'effet toxique du 5-FU 0,9 mg/ml. A partir de ces boîtes, on a prélevé des cellules de chaque souche que l'on a diluées de façon à étaler environ 200 cellules de chaque souche sur boîte de milieu complet. Par réplique sur milieu minimum + casaminoacides, on a constaté que les 5 meilleurs résistants présentaient 100 % de colonies prototrophes, tandis que la 6ème souche ne présentait que 10 % de cellules prototrophes. Parmi les 5 bons candidats, on en a gardé 2 pour continuer l'étude. Ces candidats sont appelés TGY1sp4 FUR pTG1818 clone 6 et TGY1sp4 FUR pTG1818 clone 9.

Stabilité comparée du plasmide pTG1818 dans la souche TGY1sp4 et ses dérivés FUR

Le même type d'expérience que décrit plus haut a été conduit avec TGY1sp4 pTG1818 et les deux colonies résistantes au 5-FU ; après 10 générations cellulaires en milieu complet liquide, on a dilué les cellules de chaque souche de façon à étaler 200 cellules sur boîte. 6 boîtes par souche ont été réalisées de façon à obtenir dans chaque cas environ 1200 colonies.

Après 3 jours de croissance, les colonies ont été répliquées par velours sur milieu minimum plus 0,5 % de casaminoacides. Les résultats résumés dans le tableau IV montrent une différence nette entre la souche TGY1sp4 pTG1818 qui présente environ 10 % de colonies auxotrophes par ce test, tandis que les dérivés résistant au 5-FU n'en présentent aucune.

TABLEAU IV

| | TGY1sp4 pTG1818 | | | | | |
|---|---|---|---|---|---|---|
| | Culture 1 | | Culture 2 | | Culture 3 | |
| | boîte 1 | boîte 2 | boîte 1 | boîte 2 | boîte 1 | boîte 2 |
| Nombre de colonies exigeantes pour l'uracile | 19 | 25 | 32 | 36 | 18 | 21 |
| Nombre de colonies totales | 178 | 230 | 254 | 320 | 150 | 194 |
| % de colonies auxotrophes | 10,6 | 10,8 | 12,5 | 11,0 | 12,0 | 11,8 |
| moyenne des % | $\frac{151}{1326} = 11,3 \%$ | | | | | |

| | TGY1sp4 pTG1818 [5-FU$^R$] clone 6 | | | | | |
|---|---|---|---|---|---|---|
| | Culture 1 | | Culture 2 | | Culture 3 | |
| | boîte 1 | boîte 2 | boîte 1 | boîte 2 | boîte 1 | boîte 2 |
| Nombre de colonies exigeantes pour l'uracile | 0 | 0 | 0 | 0 | 0 | 0 |
| Nombre de colonies totales | 201 | 240 | 234 | 183 | 198 | 212 |
| Total des colonies | 1268 | | | | | |

| | TGY1sp4 pTG1818 [5-FU$^R$] clone 9 | | | | | |
|---|---|---|---|---|---|---|
| | Culture 1 | | Culture 2 | | Culture 3 | |
| | boîte 1 | boîte 2 | boîte 1 | boîte 2 | boîte 1 | boîte 2 |
| Nombre de colonies exigeantes pour l'uracile | 0 | 0 | 0 | 0 | 0 | 0 |
| Nombre de colonies totales | 233 | 250 | 244 | 238 | 180 | 201 |
| Nombre total de colonies | 1346 | | | | | |

Expression de l'hirudine dans les souches TGY1sp4 pTG1818 et TGY1sp4 pTG1818 FU$^R$ clone 6

- En milieu sélectif (milieu minimum), les deux souches FU$^R$ sécrètent-elles de l'hirudine en quantité différente comparée à la production de la souche TGY1sp4 pTG1818?

On a cultivé en parallèle les souches TGY1sp4 pTG1818 et le dérivé FU$^R$clone 6 dans 10 ml de YNBG + 0,5 % de casaminoacides. L'ensemencement a été fait le matin à $DO_{700}$ 0,020 à partir de préculture d'une nuit ($DO_{700}$ 0,4 à 0,8). Les dosages d'hirudine (activité antithrombine) dans les surnageants de culture ont été réalisés aux temps 24, 48 et 72 heures. A aucun de ces trois temps on n'a trouvé de différence significative entre les deux souches (tableau V). Ceci indique que la mutation de résistance au 5-FU n'interfère pas avec le sécrétion d'hirudine.

TABLEAU V

| Temps de prélèvement | 24 h | 48 h | 72 h |
|---|---|---|---|
| Souche TGY1sp4 pTG1818<br>- $DO_{700}$<br>- Activité totale hirudine dans le surnageant (10 ml) | 4,8<br><br>75 | 7,5<br><br>128 | 8,0<br><br>131 |
| Souche TGY1sp4 pTG1818 FU$^R$ clone 6<br>- $DO_{700}$<br>- Activité totale hirudine dans le surnageant (10 ml) | 4,4<br><br>92 | 7,8<br><br>136 | 8,2<br><br>154 |

- Efficacité de sécrétion en milieu minimum + casaminoacides 0,5 % et en milieu complet.

La souche TGY1sp4 pTG1818 5-FU$^R$ clone 6 a été cultivée en parallèle en milieu minimum + casaminoacides 0,5 % et en milieu complet (100 ml de culture), et l'activité antithrombine des surnageants a été dosée aux temps 24, 48 et 72 heures.

Les résultats reportés dans le tableau VI montrent que la souche FU$^R$ sécrète pour la même masse de cellules environ 5 fois plus d'hirudine en milieu complet qu'en milieu minimum. La production maximale dans ces conditions atteint environ 10000 U/l/DO soit de l'ordre d'1 mg d'hirudine par l et par DO.

## TABLEAU VI

Souche TGYsp4 pTG1818 FU$^R$-clone 6

| Temps de prélèvement | 24 h | 48 h | 72 h |
|---|---|---|---|
| . Milieu minimum + 0.5 % de casaminoacides | | | |
| - DO$_{700}$ | 4,1 | 7,2 | 7,9 |
| - Activité totale hirudine dans le surnageant (10 ml) | 32 | 123 | 120 |
| . Milieu complet | | | |
| - DO$_{700}$ | 6 | 10 | 14 |
| - Activité totale hirudine dans le surnageant (10 ml) | 220 | 1050 | 1580 |

La souche suivante a été déposée à la Collection Nationale de Cultures de Microorganismes (CNCM) de l'Institut Pasteur, 28 rue du Docteur-Roux, 75724 PARIS CEDEX 15, en date du 30 avril 1985 :
. TGY1sp4 pTG1818 Saccharomyces cerevisiae, souche TGY1sp4 (Matα ura3-251 373-328 his-3-11-15) transformée en ura+ par un plasmide pTG1818 ; no de dépôt : I-441.

### REFERENCES

Bach, M.L., Lacroute, F., Botstein, D. (1979) Proc. Natl. Acad. Sci. USA 76,386-390.
Beggs, J.D. (1981) in Genetic Engineering vol. 2, p.175- 203, Ed. R. Williamson, Acad. Press.
Bolivar, F., Rodriguez, F.L., Greene, P.J , Betlach, M.C., Heynecker, H.L., Boyer, H.M. (1977) Gene 2, 95-113.
Broach, J.R. (1981) in the Molecular Biology of the Yeast Saccharomyces Eds. J. Strathern, E. Jones & J. Broach C.S.H. New York.
Girwitz, S.C., Boschetti, S., Rainbow, A.J., Graham, F.L. (1980) Annal Biochem. 106, p. 492.
Hinnen, A., Hicks, J.B., Finck, G.R. (1978) Proc. Natl. Acad. Sci. USA 75, 1929-1933.
Chevallier, M.R., Aigle, M. (1979) FEBS Lett. 108, 179- 180
Chevallier, M.R. Bloch, J.C., Lacroute, F. (1980) Gene 11, 11-19.
Jayaran, M., Li, Y.Y., Broach, J.R. (1983) Cell 34, 95- 104.
Jund, R., Lacroute, F. (1970) J. Bacteriol. 102, 607- 615
Jund, R., Lacroute, F. (1972) J. Bacteriol. 109, 196- 202.
Kikuchi, Y. (1983) Cell 35, 487-493.
Mortimer, R.K., Hawthorne, D.C. (1966) Genetics 53, 165- 173.
Kurjan, J. & Herskowitz, I. (1982) Cell 30, 933-943.

**Revendications**

1. Procédé de préparation d'une souche de levure transformée par un vecteur de clonage ou d'expression d'un gène codant pour une protéine déterminée qui peut être cultivée sur un milieu complet sans pression de sélection, caractérisé en ce que:
   - on bloque une première voie métabolique de l'uridine 5′-monophosphate en altérant un gène impliqué dans la synthèse d'une OMP décarboxylase active,
   - on transforme la souche par le vecteur comportant un gène codant pour une OMP décarboxylase active,
   - on bloque les autres voies métaboliques de l'uridine 5′-monophosphate en altérant au moins un gène impliqué dans la synthèse d'une UMP pyrophosphorylase active.

2. Procédé selon la revendication 1, caractérisé en ce que le gène altéré dans la première voie métabolique est le gène URA3 et en ce que le vecteur comporte l'allèle fonctionnel du gène URA3.

3. Procédé selon l'une des revendications 1 ou 2, caractérisé en ce que le gène altéré et impliqué dans la synthèse d'une UMP pyrophosphorylase active est situé dans le locus FUR1.

4. Procédé selon l'une des revendications 2 ou 3, caractérisé en ce que l'allèle fonctionnel du gène URA3 provient de l'ADN d'une souche de levure appartenant au genre Saccharomyces.

5. Procédé selon l'une des revendications 1 à 4, caractérisé en ce que le vecteur comporte au moins:
   - un fragment d'ADN codant pour une protéine déterminée sous la promotion d'un promoteur de levure,
   - l'allèle fonctionnel du gène altéré.

6. Procédé selon la revendication 5, caractérisé en ce que le vecteur comporte, en outre, une origine de réplication dans les levures.

7. Procédé selon la revendication 6, caractérisé en ce que l'origine de réplication est celle du plasmide 2 μ de levure.

8. Procédé selon l'une des revendications 1 à 7, caractérisé en ce que le gène codant pour une protéine déterminée est le gène xy1E.

9. Procédé selon la revendication 8, caractérisé en ce que le plasmide vecteur est pTG843 déposé dans la souche TGY8-3 à la CNCM sous le n° I-326.

10. Procédé selon l'une des revendications 1 à 9, caractérisé en ce qu'on transforme une souche de levure ura3 par un vecteur complémentant l'auxotrophie et on sélectionne les souches ura⁺ puis on bloque les autres voies métaboliques des souches ura⁺.

11. Procédé selon la revendication 10, caractérisé en ce que, pour bloquer les autres voies métaboliques des souches ura⁺, on croise ces souches avec un mutant fur1 et on sélectionne les produits de croisement ura⁺, fur1.

12. Procédé selon la revendication 10, caractérisé en ce que, pour bloquer les autres voies métaboliques des souches ura⁺, on sélectionne les mutants spontanés ura⁺, fur1.

13. Procédé selon l'une des revendications 1 à 9, caractérisé en ce qu'on transforme une souche diploïde homozygote pour ura3 et hétérozygote pour fur1.

14. Procédé selon la revendication 13, caractérisé en ce qu'on sélectionne les ségrégants haploïdes fur1 après sporulation d'une souche diploïde selon la revendication 13.

15. Procédé selon la revendication 13, caractérisé en ce qu'on sélectionne, après conversion génique d'une souche diploïde selon la revendication 13, les dérivés homozygotes pour fur1.

16. Levure préparée par un procédé selon l'une des revendications 1 à 15.

17. Levure selon la revendication 16, caractérisée en ce qu'il s'agit d'une souche de levure appartenant au genre Saccharomyces, notamment Saccharomyces cerevisiae.

18. Souche de Saccharomyces caractérisée en ce que:
   - elle est incapable de synthétiser une OMP décarboxylase active,
   - elle est incapable de synthétiser une UMP pyrophosphorylase active, et
   - elle est transformée par un plasmide d'expression d'une protéine déterminée portant, en outre, un gène codant pour une OMP décarboxylase active.

19. Souche selon la revendication 18, caractérisée en ce que la protéine déterminée est la catéchol oxygénase.

20. Souche selon la revendication 18, caractérisée en ce que la protéine déterminée est l'alpha-1-antitrypsine.

21. Souche selon la revendication 18, caractérisée en ce que la protéine déterminée est l'hirudine.

22. Procédé de préparation d'une protéine déterminée, caractérisé en ce qu'on cultive sur un milieu complet une souche de cellule selon l'une des revendications 16 à 21 et en ce qu'on récupère la protéine obtenue.

**Claims**

1. A method for preparing a yeast strain transformed by a vector for cloning or expression of a gene which codes for a specified protein, which strain can be cultured on a complete medium without selection pressure, characterized in that:

– a first metabolic pathway of uridine 5'-monophosphate is blocked by altering a gene involved in the synthesis of an active OMP decarboxylase,

– the strain is transformed with the vector which contains a gene coding for an active OMP decarboxylase,

– the other metabolic pathways of uridine 5'-monophosphate are blocked by altering at least one of the genes involved in the synthesis of an active UMP pyrophosphorylase.

2. A method as claimed in claim 1, characterized in that the altered gene in the first metabolic pathway is the URA3 gene and the vector contains the functional allele of the URA3 gene.

3. A method as claimed in claim 1 or 2, characterized in that the altered gene which is involved in the synthesis of an active UMP pyrophosphorylase is situated in the FUR1 Locus.

4. A method as claimed in claims 2 or 3, characterized in that the functional allele of the URA3 gene originates from the DNA of a yeast strain belonging to the genus Saccharomyces.

5. A method as claimed in anyone of claims 1 to 4, characterized in that the vector contains at least:

– a DNA fragment which codes for a specified protein under the control of a yeast promoter,

– the functional allele of the altered gene.

6. A method as claimed in claim 5, characterized in that the vector contains in addition an origin of replication in yeasts.

7. A method as claimed in claim 6, characterized in that the origin of replication is that of the 2 μ plasmid of yeasts.

8. A method as claimed in anyone of claims 1 to 7, wherein the gene which codes for a specified protein is the xy1E gene.

9. A method as claimed in claim 8, characterized in that the vector plasmid is pTG843 deposited in the strain TGY8-3 with the CNCM under n° I-326.

10. A method as claimed in anyone of claims 1 to 9, characterized in that a ura3 yeast strain is transformed with a vector which complements the auxotrophy, the ura+ strains are selected and then the other metabolic pathways of the ura+ strains are blocked.

11. A process as claimed in claim 10, characterized in that to block the other metabolic pathways of the ura+ strains, these strains are crossed with a fur1 mutant and the ura+, fur1 cross products are selected.

12. A method as claimed in claim 10, characterized in that, to block the other metabolic pathways of the ura+ strains, the spontaneous ura+, fur1 mutants are selected.

13. A method as claimed in anyone of claims 1 to 9, characterized in that a diploid strain which is homozygous for ura3 and heterozygous for fur1 is transformed.

14. A method as claimed in claim 13, characterized in that the fur1 haploid segregants are selected after sporulation of a diploid strain as claimed in claim 13.

15. A method as claimed in claim 13, characterized in that the derivatives which are homozygous for fur1 are selected after gene conversion of a diploid strain as claimed in claim 13.

16. Yeast cell prepared by a method as claimed in anyone of claims 1 to 15.

17. A yeast cell as claimed in claim 20, which is a yeast strain belonging to the genus Saccharomyces in particular Saccharomyces cerevisiae.

18. A Saccharomyces strain characterized in that:

– it is incapable of synthesizing an active OMP decarboxylase,

– it is incapable of synthesizing an active UMP pyrophosphorylase, and

– it is transformed by a plasmid for expression of a specified protein, carrying in addition a gene which codes for an active OMP decarboxylase.

19. A strain as claimed in claim 18, characterized in that the specified protein is catechol oxygenase.

20. A strain as claimed in claim 18, characterized in that the specified protein is ₁-antritrypsin.

21. A strain as claimed in claim 18, characterized in that the specified protein is hirudin.

22. A method for preparing a specified protein, characterized in that a cell strain as claimed in anyone of claims 16 to 21 is cultured on a complete medium, and wherein the protein obtained is recovered.

## Patentansprüche

1. Verfahren zur Herstellung eines Hefepilz-Stammes, der durch einen Clonierungs- oder Expressionsvektors eines Gens, das für ein vorgegebenes Protein codiert, transformiert worden ist, der auf einem vollständigen Milieu ohne Selektionsdruck kultiviert werden kann, dadurch gekennzeichnet, daß man

- eine erste Stoffwechselbahn des Uridin-5'-monophosphats blockiert durch Änderung eines Gens, das an der Synthese einer aktiven OMP-Decarboxylase beteiligt ist,

- den Stamm durch den Vektor, der ein Gen trägt, das für eine aktive OMP-Decarboxylase codiert, transformiert und

- die anderen Stoffwechselbahnen des Uridin-5'-monophosphats blockiert durch Änderung mindestens eines Gens, das an der Synthese einer aktiven UMP-Pyrophosphorylase beteiligt ist.

2. Verfahren nach Anspruch 1, dadurch gekennzeichnet, daß es sich bei dem in der ersten Stoffwechselbahn geänderten Gen um das Gen URA3 handelt und daß der Vektor das funktionelle Allel des Gens URA3 enthält.

3. Verfahren nach Anspruch 1 oder 2, dadurch gekennzeichnet, daß das geänderte und an der Synthese einer aktiven UMP-Pyrophosphorylase beteiligte Gen an der Stelle FUR1 angeordnet ist.

4. Verfahren nach Anspruch 2 oder 3, dadurch gekennzeichnet, daß das funktionelle Allel des Gens URA3 von der DNA eines Hefepilz-Stammes stammt, der zum Genus Saccharomyces gehört.

5. Verfahren nach einem der Ansprüche 1 bis 4, dadurch gekennzeichnet, daß der Vektor mindestens enthält:

- ein DNA-Fragment, das für ein vorgegebenes Protein codiert unter der Promotion eines Hefepilz-Promotors und

- das funktionelle Allel des geänderten Gens.

6. Verfahren nach Anspruch 5, dadurch gekennzeichnet, daß der Vektor außerdem einen Replikationsursprung in den Hefepilzen aufweist.

7. Verfahren nach Anspruch 6, dadurch gekennzeichnet, daß der Replikationsursprung derjenige des Hefepilz-Plasmids 2 μ ist.

8. Verfahren nach einem der Ansprüche 1 bis 7, dadurch gekennzeichnet, daß es sich bei dem Gen, das für ein vorgegebenes Protein codiert, um das Gen xy1E handelt.

9. Verfahren nach Anspruch 8, dadurch gekennzeichnet, daß es sich bei dem Plasmid-Vektor um pTG843 handelt, der in dem Stamm TGY8-3 bei der CNCM unter der Nummer I-326 hinterlegt ist.

10. Verfahren nach einem der Ansprüche 1 bis 9, dadurch gekennzeichnet, daß man einen Hefepilz-Stamm ura3 durch einen die Auxotrophie vervollständigenden Vektor transformiert und die Stämme ura+ selektiert und dann die anderen Stoffwechselbahnen der Stämme ura+ blockiert.

11. Verfahren nach Anspruch 10, dadurch gekennzeichnet, daß zum Blockieren der anderen Stoffwechselbahnen der Stämme ura+ man diese Stämme mit einer Mutante fur1 kreuzt und die Kreuzungsprodukte ura+, fur1 selektioniert.

12. Verfahren nach Anspruch 10, dadurch gekennzeichnet, daß man zum Blockieren der anderen Stoffwechselbahnen der Stämme ura+ die spontanen Mutanten ura+, fur1 selektioniert.

13. Verfahren nach einem der Ansprüche 1 bis 9, dadurch gekennzeichnet, daß man einen diploiden Homozygoten-Stamm für ura 3 und einen diploiden Heterozygoten-Stamm für fur1 transformiert.

14. Verfahren nach Anspruch 13, dadurch gekennzeichnet, daß man die haploiden Segreganten fur1 nach der Sporenbildung eines diploiden Stammes gemäß Anspruch 13 selektioniert.

15. Verfahren nach Anspruch 13, dadurch gekennzeichnet, daß man nach der genetischen Umwandlung eines diploiden Stammes nach Anspruch 13 die homozygoten Derivate für fur1 selektioniert.

16. Hefepilz, hergestellt nach einem Verfahren nach einem der Ansprüche 1 bis 15.

17. Hefepilz nach Anspruch 16, dadurch gekennzeichnet, daß es sich dabei um einen Hefepilz-Stamm handelt, der zum Genus Saccharomyces, insbesondere Saccharomyces cerevisiae, gehört.

18. Stamm von Saccharomyces, dadurch gekennzeichnet, daß er

- nicht in der Lage ist, eine aktive OMP-Decarboxylase zu synthetisieren,

- nicht in der Lage ist, eine aktive UMP-Pyrophosphorylase zu synthetisieren und

- durch ein Expressionsplasmid eines vorgegebenen Proteins, das außerdem ein Gen trägt, das für eine aktive OMP-Decarboxylase codiert, transformiert wird.

19. Stamm nach Anspruch 18, dadurch gekennzeichnet, daß es sich bei dem vorgegebenen Protein um die Brenzcatechin-Oxygenase handelt.

20. Stamm nach Anspruch 18, dadurch gekennzeichnet, daß es sich bei dem vorgegebenen Protein um das $\alpha_1$-Antitrypsin handelt.

21. Stamm nach Anspruch 18, dadurch gekennzeichnet, daß es sich bei dem vorgegebenen Protein um das Hirudin handelt.

22. Verfahren zur Herstellung eines vorgegebenen Proteins, dadurch gekennzeichnet, daß man einen Zellenstamm nach einem der Ansprüche 16 bis 21 auf einem vollständigen Milieu kultiviert und das erhaltene Protein abtrennt.

FIG-1

ptg 964

1- Bgl II
2- Pst I

ptg 849

1- Pst I
2- Bgl II
3- Phosphatase

LIGASE

FIG-2

ptg 968

EP 0 173 619 B1

FIG-3

FIG-4

4α                    4β

TEST QUALITATIF DE PRODUCTION
DE LA β-LACTAMASE

EP 0 173 619 B1